Europäisches Patentamt

European Patent Office

Office européen des brevets

(11) Publication number: **O O13 600**
**B1**

## (12) EUROPEAN PATENT SPECIFICATION

(45) Date of publication of patent specification: **11.04.84**

(21) Application number: **80300026.4**

(22) Date of filing: **04.01.80**

(51) Int. Cl.³: **C 07 C 31/20,**
**C 07 C 33/025,**
**C 07 C 29/38**

(54) **Production of alkenols and alkanediols by condensation of aldehyde and ethylenically unsaturated compounds.**

(30) Priority: **05.01.79 US 1124**

(43) Date of publication of application:
**23.07.80 Bulletin 80/15**

(45) Publication of the grant of the patent:
**11.04.84 Bulletin 84/15**

(84) Designated Contracting States:
**BE DE FR GB IT NL**

(56) References citeD:
**DE - A - 1 901 142**
**GB - A - 590 571**
**US - A - 3 414 588**
**US - A - 3 702 886**
**US - A - 3 709 979**
**US - A - 4 076 842**

(73) Proprietor: **MOBIL OIL CORPORATION**
**150 East 42nd Street**
**New York New York 10017 (US)**

(72) Inventor: **Chang, Clarence Dayton**
**11 Murray Place**
**Princeton, New Jersey 08540 (US)**
Inventor: **Lang, William Harry**
**Box 245 A RD1 Mount Rose Road**
**Pennington, New Jersey 08534 (US)**
Inventor: **Morgan, Norman James**
**12 Landowne Road**
**Burlington, New Jersey 08016 (US)**

(74) Representative: **West, Alan Harry**
**Mobil Court 3 Clements Inn**
**London WC2A 2EB (GB)**

Courier Press, Leamington Spa, England.

Production of alkenols and alkanediols by condensation of aldehyde and ethylenically unsaturated compounds

This invention relates to selective production of alkenols and alkanediols utilizing a specified crystalline aluminosilicate zeolite-containing catalyst.

It has heretofore been known to conduct Prins-type condensation reactions, i.e. condensation between an aldehyde and an ethylenically unsaturated compound, in the presence of mineral acid catalysts such as sulfuric acid or phosphoric acid. The products so obtained, as described by Arundale et al. in Chemical Reviews 51, 505—517 (1952), are dioxanes, contrasted with the alkenols and alkanediols obtained in accordance with the process of the present invention. Thus, condensation of formaldehyde and isobutene in the presence of a catalyst of aqueous sulfuric acid produced 4,4-dimethyl-m-dioxane. Similar dioxane products are obtained with catalysts of large pore crystalline aluminosilicate zeolites of the faujasite type, as described in U.S. 3,414,588. The reactions effected with the above catalysts are non-shape selective as compared with those encountered in the process of the present invention wherein alkenols and alkanediols are obtained as the major product utilizing a particularly defined crystalline aluminosilicate zeolite-containing catalyst. In addition, the process described herein, as contrasted with that described in U.S. 3,414,588, affords, under similar conditions, the production of bath alkanediols and alkenols.

It has also heretofore been known to react an unsaturated compound with a aldehyde in the presence of a Friedel-Crafts type catalyst as described, for example, in British Patent No. 1,205,762. Utilizing such type catalyst, isobutene may be reacted with formaldehyde to yield 3-methyl-3-buten-1-ol. In contrast to the specified crystalline aluminosilicate zeolite-containing catalysts employed in the process of the present invention, the Friedel-Crafts type catalysts which have been previously used are water sensitive and are potential pollutants giving rise to disposal problems.

In accordance with the present invention, there has been discovered a process for selectively producing an alcohol product constituting alkenols and alkanediols, without encountering the inherent problems of the prior art, by effecting a condensation reaction between formaldehyde or copolymer thereof and an aliphatic monoolefinically unsaturated compound under controlled conditions of temperature in the presence of water and a particular class of crystalline aluminosilicate zeolites characterized by a silica to alumina mole ratio of at least 12 and a constraint index within the range of 1 to 12.

The alkenols so obtained are useful intermediates for the production of diene monomers, glycols and other valuable chemicals. Thus, the reaction between isobutene and formaldehyde yield products, i.e. 3-methyl-3-buten-1-ol and 3-methylbutanediols which are particularly valuable as isoprene precursors.

The invention described herein contemplates effecting Prins-type reactions of a selective nature between formaldehyde and certain unsaturated compounds to yield alkenols and alkanediols in the presence of a catalyst comprising a crystalline aluminosilicate zeolite having a silica to alumina mole ratio of at least 12 and a constraint index within the range of 1 to 12.

In particular, this invention is directed to the production of alkenols and alkanediols containing 3 to 25 carbon atoms by effecting reaction between formaldehyde or polymer thereof and an aliphatic monoolefinically unsaturated compound containing 2 to 24 and preferably 3 to 8 carbon atoms in the presence of water and the aforenoted catalyst. Generally, it is contemplated that the alcohol product produced in accordance with the present process will contain between 4 and 9 carbon atoms.

The unsaturated compounds utilized will usually contain 2 to 24 carbon atoms and include aliphatic compounds that have a double bond between two carbon atoms within their molecular moieties. In addition, these ethylenically unsaturated compounds have a hydrogen atom that is bonded alpha to the carbon to carbon ethylenic linkage. Exemplary of these compounds are olefins such as ethylene, propylene, isobutene, 2-pentene, diisobutylene and trimethylethylene. In addition, derivatives of these compounds containing substituents such as alkyl, alkoxy, the halogens, cyano, hydroxy and other groups may be employed.

The aldehyde used in the present process is formaldehyde. In addition, polymers such as paraformaldehyde which will decompose under the reaction conditions of the present process to produce formaldehyde, may be used. It is contemplated that formaldehyde may be employed in aqueous solution or with an inert organic solvent. Thus, formaldehyde may be conveniently introduced into the present process by the use of formalin, an aqueous solution containing 20 to 50 percent of formaldehyde.

In accordance with the present invention, it has been found that alkenols and alkanediols may be produced as primary reaction products utilizing a catalyst comprising a crystalline aluminosilicate zeolite having a silica to alumina ratio of at least about 12 and a constraint index within the approximate range of 1 to 12. For example, employing such type catalyst for effecting reaction between isobutene and formaldehyde, 3-methyl butenols and 3-methylbutanediols are obtained. These compounds are valuable intermediates in the production of isoprene and are to be contrasted with the major condensation product of 4,4-dimethyl-m-dioxane obtained when the above reaction is catalyzed by mineral acids or crystalline aluminosilicate zeolites of the faujasite type.

The zeolites employed herein are members of a novel class of zeolites exhibiting some unusual properties. These zeolites induce profound transformation of aliphatic hydrocarbons in commercially desirable yields and are generally highly effective in conversion reactions involving aromatic hydrocarbons. Although they have unusually low alumina contents, i.e., high silica to alumina ratios, they are very active even when the silica to alumina ratio exceeds 30. The activity is surprising since catalyst activity is generally attributed to framework aluminum atoms and cations associated with these aluminum atoms. These zeolites retain their crystallinity for long periods in spite of the presence of steam at high temperature which induces irreversible collapse of the framework of other zeolites, e.g., of the X and A type.

An important characteristic of the crystal structure of this class of zeolites is that it provides constrained access to, and egress from the intracrystalline free space by virtue of having a pore dimension greater than about 5 Angstroms and pore windows of about a size such as would be provided by 10-membered rings of oxygen atoms. It is to be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. Briefly, the type of zeolites used in this invention possess, in combination: a silica to alumina mole ratio of at least 12 and a structure providing constrained access to the crystalline free space.

The silica to alumina ratio referred to may be determined by conventional analysis. This ratio is meant to represent as closely as possible, the ratio in the rigid anionic framework of the zeolite crystal and to exclude aluminum in the binder or in cationic or other form within the channels. Although zeolites with a silica to alumina ratio of at least 12 are useful, it is preferred to use zeolites having higher ratios of at least about 30. Such zeolites, after activation, acquire an intracrystalline sorption capacity for normal hexane which is greater than that for water, i.e., they exhibit "hydrophobic" properties. It is believed that this hydrophobic character is advantageous in the present invention.

The type zeolites useful in this invention freely sorb normal hexane and have a pore dimension greater than about 5 Angstroms. In addition, the structure must provide constrained access to larger molecules. It is sometimes possible to judge from a known crystal structure whether such constrained access exists. For example, if the only pore windows in a crystal are formed by 8-membered rings of oxygen atoms, then access to molecules of larger cross-section than normal hexane is excluded and the zeolite is not of the desired type. Windows of 10-membered rings are preferred, although, in some instances, excessive puckering or pore blockage may render these zeolites ineffective. Twelve-membered rings do not generally appear to offer sufficient constraint to produce the advantageous conversions although puckered structures exist such as TMA Offretite which is a known effective zeolite. Also, structures can be conceived, due to pore blockage or other cause, that may be operative.

Rather than attempt to judge from crystal structure whether or not a zeolite possesses the necessary constrained access, a simple determination of the "constraint index" may be made by passing continuously a mixture of an equal weight of normal hexane and 3-methylpentane over a sample of zeolite at atmospheric pressure according to the following procedure. A sample of the zeolite, in the form of pellets or extrudate, is crushed to a particle size about that of coarse sand and mounted in a glass tube. Prior to testing, the zeolite is treated with a stream of air at 537.7°C (1000°F) for at least 15 minutes. The zeolite is then flushed with helium and the temperature adjusted between 287.7°C (550°F) and 510°C (950°F) to give an overall conversion between 10% and 60%. The mixture of hydrocarbons is passed at 1 liquid hourly space velocity (i.e., 1 volume of liquid hydrocarbon per volume of zeolite per hour) over the zeolite with a helium dilution to give a helium to total hydrocarbon mole ratio of 4:1. After 20 minutes on stream, a sample of the effluent is taken and analyzed, most conveniently by gas chromatography, to determine the fraction remaining unchanged for each of the two hydrocarbons.

The "constraint index" is calculated as follows:

$$\text{Constraint Index} = \frac{\log_{10}(\text{fraction of n-hexane remaining})}{\log_{10}(\text{fraction of 3-methylpentane remaining})}$$

The constraint index approximates the ratio of the cracking rate constants for the two hydrocarbons. Zeolites suitable for the present invention are those having a constraint index in the approximate range of 1 to 12. Constraint Index (CI) values for some typical zeolites are:

3

# 0 013 600

| CAS | C.I. |
|---|---|
| ZSM—5 | 8.3 |
| ZSM—11 | 8.7 |
| ZSM—12 | 2 |
| ZSM—23 | 9.1 |
| ZSM—38 | 2 |
| ZSM—35 | 4.5 |
| Clinoptilolite | 3.4 |
| TMA Offretite | 3.7 |
| Beta | 0.6 |
| ZSM—4 | 0.5 |
| H-Zeolon | 0.4 |
| REY | 0.4 |
| Amorphous Silica-Alumina | 0.6 |
| Erionite | 38 |

It is to be realized that the above constraint index values typically characterize the specified zeolites but that such are the cumulative result of several variables used in determination and calculation thereof. Thus, for a given zeolite depending on the temperature employed within the aforenoted range of 260°C (500°F) to 510°C (950°F), with accompanying conversion between 10% and 60%, the constraint index may vary within the indicated approximate range of 1 to 12. Likewise, other variables such as the crystal size of the zeolite, the presence of possible occluded contaminants and binders intimately combined with the zeolite may affect the constraint index. It will accordingly be understood that those skilled in the art that the constraint index, as utilized herein, while affording a highly useful means for characterizing the zeolites of interest is approximate, taking into consideration the manner of its determination; with probability, in some instances, of compounding variable extremes.

While the above experimental procedure will enable one to achieve the desired overall conversion of 10 to 60% for most catalyst samples and represents preferred conditions, it may occasionally be necessary to use somewhat more severe conditions for samples of very low activity, such as those having a high silica to alumina ratio. In those instances, a temperature of up to about 537.7°C (1000°F) and a liquid hourly space velocity of less than one, such as 0.1 or less, can be employed in order to achieve a minimum conversion of about 10%.

The class of zeolites defined herein is exemplified by ZSM—5, ZSM—11, ZSM—12, ZSM—23, ZSM—35 and ZSM—38 and other similar materials. U.S. Patent 3,702,886 describing and claiming ZSM—5 is incorporated herein by reference.

ZSM—11 is more particularly described in U.S. Patent 3,709,979, the entire contents of which are incorporated herein by reference.

ZSM—12 is more particularly described in U.S. Patent 3,832,449, the entire contents of which is incorporated herein by reference.

ZSM—23 is more particularly described in U.S. 4,076,842, the entire contents of which is incorporated by reference.

ZSM—35 is more particularly described in U.S. Patent 4,016,245 the entire contents of which is incorporated herein by reference.

ZSM—38 is more particularly defined in U.S. Patent 4,046,859, the entire contents of which is incorporated herein by reference.

The specific zeolites described, when prepared in the presence of organic cations, are catalytically inactive, possibly because the intracrystalline free space is occupied by organic cations from the forming solution. They may be activated by heating in an inert atmosphere at 37.7°C (100°F) for one hour, for

4

example, followed by base exchange with ammonium salts followed by calcination at 537.7°C (1000°F) in air. The presence of organic cations in the forming solution may not be absolutely essential to the formation of this type zeolite; however, the presence of these cations does appear to favor the formation of this special type of zeolite. More generally, it is desired to activate this type catalyst by base exchange with ammonium salts followed by calcination in air at about 537.7°C (1000°F) for from about 15 minutes to about 24 hours.

Natural zeolites may sometimes be converted to this type zeolite catalyst by various activation procedures and other treatments such as base exchange, steaming, alumina extraction and calcination, in combinations. Natural minerals which may be so treated include ferrierite, brewsterite, stilbite, dachiardite, epistilbite, heulandite, and clinoptilolite. The preferred crystalline aluminosilicates are ZSM—5, ZSM—11, ZSM—12, ZSM—23, ZSM—38 and ZSM—35 with ZSM—5 and ZSM—11 particularly preferred. ZSM—11 appears to be somewhat more selective than ZSM—5 for producing enol and diol.

In a preferred aspect of this invention, the zeolites hereof are selected as those having a crystal framework density, in the dry hydrogen form, of not substantially below about 1.6 grams per cubic centimeter. Therefore, the preferred zeolites of this invention are those having a constraint index as defined above of 1 to 12 and a dried crystal density of not less than about 1.6 grams per cubic centimeter. The dry density for known structures may be calculated from the number of silicon plus aluminum atoms per 1000 cubic Angstroms, as given, e.g., on Page 19 of the article on Zeolite Structure by W. M. Meier. This paper, the entire contents of which are incorporated herein by reference, is included in "Proceedings of the Conference on Molecular Sieves, London, April, 1967," published by the Society of Chemical Industry, London, 1968. When the crystal structure is unknown, the crystal framework density may be determined by classical pyknometer techniques. For example, it may be determined by immersing the dry hydrogen form of the zeolite in an organic solvent which is not sorbed by the crystal. It is possible that the unusual sustained activity and stability of this class of zeolites is associated with its high crystal anionic framework density of not less than about 1.6 grams per cubic centimeter. This high density, of course, must be associated with a relatively small amount of free space within the crystal, which might be expected to result in more stable structures. This free space, however, is important as the locus of catalytic activity.

Crystal framework densities of some typical zeolites are:

| Zeolite | Void Volume | Framework Density |
|---|---|---|
| Ferrierite | 0.28 cc/cc | 1.76 g/cc |
| Mordenite | .28 | 1.7 |
| ZSM—5, —11 | .29 | 1.79 |
| Dachiardite | .32 | 1.72 |
| L | .32 | 1.61 |
| Clinoptilolite | .34 | 1.71 |
| Laumontite | .34 | 1.77 |
| ZSM—4 (Omega) | .38 | 1.65 |
| Heulandite | .39 | 1.69 |
| P | .41 | 1.57 |
| Offretite | .40 | 1.55 |
| Levynite | .40 | 1.54 |
| Erionite | .35 | 1.51 |
| Gmelinite | .44 | 1.46 |
| Chabazite | .47 | 1.47 |
| A | .5 | 1.3 |
| Y | .48 | 1.27 |

When synthesized in the alkali metal form, the zeolite is conveniently converted to the hydrogen form, generally by intermediate formation of the ammonium form as a result of ammonium ion exchange and calcination of ammonium form to yield the hydrogen form. In addition to the hydrogen form, other forms of the zeolite wherein the original alkali metal has been reduced to less than about 1.5 per cent by weight may be used. Thus, the original alkaki metal of the zeolite may be replaced by ion exchange with other suitable ions of Groups IB to VIII of the Periodic Table, including, by way of example, nickel, copper, zinc, palladium, calcium or rare earth metals.

Generally, however, the zeolite either directly or via initial ammonium exchange followed by calcination, is preferably hydrogen exchanged such that a predominate proportion of its exchangeable cations are hydrogen ions. In general, it is contemplated that more than 50 percent and preferably more than 75 percent of the cationic sites of the crystalline aluminosilicate zeolite will be occupied by hydrogen ions.

In practicing the desired condensation process, it may be desirable to incorporate the above-described crystalline aluminosilicate zeolite in another material resistant to the temperature and other conditions employed in the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays, which can be composited with the zeolite include those of the montmorillonite and kaolin families, which families includes the sub-bentonites and the kaolins commonly known as Dixie, McNamee-Georgia and Florida clays or others in which the main mineral constituent is halloysite, kaolinite, dickite, nacrite or anauxite. Such clays can be used in a raw state as originally mined or initially subjected to calcination, acid treatment or chemical modification.

In addition to the foregoing materials, the zeolites employed herein may be composited with a porous matrix material, such as alumina, silica-alumina, silica-magnesia, silica-zirconia, silica-thoria, silica-berylia, silica-titania as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix may vary widely with the zeolite content ranging from between about 1 to about 99 percent by weight and more usually in the range of about 5 to 80 percent by weight of the composite.

The temperature at which the reaction described herein is carried out generally does not exceed about 250°C. At higher temperatures, undesirable side reactions such as degradation and polymerization of the unsaturated compounds are encountered which reduce the yield of products as well as the activity of the catalyst. While the particular reaction temperature employed will depend on the reactants undergoing condensation and the specific catalyst used, such temperature is usually between about 20°C and about 200°C and preferably between about 50°C and about 150°C.

The pressure at which the present process is conducted may range from about atmospheric to considerably higher pressures. Generally, the pressure is autogeneous, i.e., the vapor pressure of the reactants, condensation products and solvents at the reaction temperature of the process. Usually, the pressure employed will range from atmospheric to about 500 psig and preferably between about 100 and about 200 psig.

The molar ratio of reactants, i.e. unsaturated compound and formaldehyde, used may vary over wide limits. In general, the molar ratio of unsaturated compounds to formaldehyde will be within the approximate range of 0.25 to 15 and more usually between about 0.4 and about 10. As a practical matter, it has been found that unwanted polymerization of the unsaturated compound can be minimized by initial contact of the catalyst with the formaldehyde reactant followed by introduction of the unsaturated compound reactant.

Where the reactants employed are normally solid or highly volatile, the reaction is desirably carried out in an aqueous medium or in the presence of an inert solvent. The latter is preferably nonvolatile and non-reactive under the conditions employed for effecting the desired condensation between formaldehyde and unsaturated compound. Representative inert solvents include petroleum ether, benzene, ethanol, acetone, methanol, dioxane, chloroform and methylenechloride.

The following examples will serve to illustrate the process of this invention without limiting the same:

Example 1

Isobutene (28.6 grams) and aqueous 37% formaldehyde (81.2 grams) were charged to a stirred autoclave, along with powdered HZSM—11 (4.8 grams). The autoclave was heated with stirring to 67°—68°C and held for 5 1/2 hours. The temperature was then raised to 102°C for an additional 2 hours. Pressure was autogenic. Samples were taken during this period and analyzed. Results are given in Table I below:

TABLE I

| | | | | |
|---|---|---|---|---|
| Temperature, °C | 68 | 67 | 67 | 102 |
| Pressure, kPa | 1066 | 997 | 997 | 1066 |
| Time, hr. | 1 | 3 1/4 | 5 1/4 | 7 |
| CONVERSION, % | 40.4 | 51.5 | 49.7 | 58.2 |
| PRODUCTS (a), wt. % | | | | |
| 3-Methylbutenols | 44.2 | 41.7 | 32.4 | 29.5 |
| 3-Methylbutanediols | 38.6 | 44.7 | 45.4 | 50.3 |
| 4,4-Dimethyl-1,3-dioxane | 11.5 | 9.3 | 10.5 | 13.4 |
| High molecular weight compounds | 5.7 | 4.3 | 11.7 | 6.8 |
| SELECTIVITY (b), wt. % | 82.8 | 86.4 | 77.8 | 79.8 |

(a) t-Butanol considered as "unconverted" i-butene

(b) Selectivity = (enols + diols) × 100/Total product

Example 2

Isobutene (28.6 grams) and aqueous 37% formaldehyde (81.2 grams) were charged to a stirred autoclave, along with powdered HZSM—5 (8.3 grams). The autoclave was heated with stirring to 66°—67°C. Samples were taken over a 2 hour period, and gave the analyses shown in Table II below:

TABLE II

| | | | | |
|---|---|---|---|---|
| Temperature, °C | 66 | 66 | 66 | 67 |
| Pressure, kPa | 1066 | 1032 | 1032 | 1032 |
| Time, hr. | 1/2 | 3/4 | 1 | 2 |
| CONVERSION, % | 30.7 | 36.2 | 43.7 | 49.2 |
| PRODUCTS 5(a), % | | | | |
| 3-Methylbutenols | 50.2 | 48.3 | 40.3 | 35.0 |
| 3-Methylbutanediols | 26.2 | 35.2 | 34.9 | 35.9 |
| 4,4-Dimethyl-1,3-dioxane | 16.6 | 13.5 | 14.3 | 13.8 |
| High molecular weight compounds | 7.0 | 3.0 | 10.5 | 15.3 |
| SELECTIVITY (b), wt. % | 76.4 | 83.5 | 75.2 | 70.9 |

(a) t-Butanol considered as "unconverted" i-butene

(b) Selectivity = (enols + diols) × 100/Total product

**0 013 600**

Example 3

In this example, zeolites ZSM—5, ZSM—11 and ZSM—23 are compared with zeolite REY for the production of methylbutenols and methylbutanediols from isobutene and formaldehyde. In each run, the catalyst was charged to a tubular flow reactor and a mixture of formalin solution and isobutene (containing 20 mole % formaldehyde, 5 mole % methanol, 48 mole % water and 26 mole % isobutene) was passed over the catalyst at 248°C, 3548 kPa and 1.4 LHSV. The results, given in Table III below, indicate that the ZSM—5, ZSM—11 and ZSM—23 catalysts exhibit a greater selectivity to the methyl-butenol and methylbutanediol products than does the REY catalyst.

TABLE III

| Catalyst* | ZSM—5 | ZSM—11 | ZSM—23 | REY |
|---|---|---|---|---|
| **Conditions** | | | | |
| Temperature °C | 251 | 250 | 251 | 248 |
| Pressure kPa | 3548 | 3548 | 3548 | 3548 |
| LHSV | 1.4 | 1.4 | 1.4 | 1.4 |
| Time on Stream, days | 1 | 1 | .9 | .8 |
| *Conversion of Isobutene*, % | 15 | 23 | 22 | 23 |
| *Products Distribution*[a] Mole % | | | | |
| 3-Methylbutenols | 38.8 | 32.3 | 38.1 | 22.0 |
| 3-Methylbutanediols | 33.3 | 38.2 | 31.5 | 26.0 |
| 4,4-Dimethyl-1,3-dioxane | 22.2 | 17.6 | 17.1 | 42.8 |
| High Molecular Weight Compounds | 5.5 | 11.7 | 13.5 | 9.0 |
| *Selectivity*[b] Mole % | 72 | 70.5 | 69.6 | 48 |

*Zeolite catalysts bound with about 33%, by weight, alumina

[a] t-Butanol considered as "unconverted" i-butene

[b] Selectivity = (enols + diols) × 100/Total product

**Claims**

1. A process for producing an alcohol product containing 3 to 25 carbon atoms comprising an alkenol and an alkanediol which comprises effecting reaction between formaldehyde and an aliphatic monoolefinically unsaturated compound containing from 2 to 24 carbon atoms at a temperature not exceeding 250°C in the presence of water and a catalyst comprising a crystalline aluminosilicate zeolite characterized by a silica to alumina mole ratio of at least 12 and a constraint index within the range of 1 to 12 and recovering a condensation product constituting said alcohol product.

2. The process of Claim 1 wherein said alcohol product contains 4 to 9 carbon atoms and said unsaturated compound contains from 3 to 8 carbon atoms.

3. The process of claim 1 or 2 wherein said alcohol product contains 5 carbon atoms and said unsaturated compound contains 4 carbon atoms.

4. The process of any preceding claim wherein said alcohol product consists of 3-methylbutenols and 3-methylbutanediols and said unsaturated compound is isobutene.

8

5. The process of any preceding claim wherein said unsaturated compound has a hydrogen atom that is bonded alpha to the carbon to carbon ethylenic linkage.

6. The process of any preceding claim wherein said temperature is between 20°C and 200°C, and preferably between 50°C and 150°C.

7. The process of any preceding claim wherein the formaldehyde is in the form of an aqueous solution.

8. The process of any preceding claim wherein said crystalline aluminosilicate zeolite is ZSM—5, ZSM—11 or ZSM—23.

9. The process of any preceding claim wherein said crystalline aluminosilicate zeolite is admixed with a binder therefor.

10. The process of any preceding claim wherein said reaction takes place in an inert solvent medium.

11. The process of any preceding claim wherein said reaction is effected at pressures from atmospheric to superatmospheric.

## Revendications

1. Un procédé pour la production d'un produit de type alcool contenant 3 à 25 atomes de carbone comprenant un alcénol et un alcanediol qui consiste à effectuer une réaction entre le formaldéhyde et un composé aliphatique à insaturation monooléfinique contenant 2 à 24 atomes de carbone à une température ne dépassant pas 250°C, en présence d'eau et d'un catalyseur comprenant un aluminosilicate cristalline zéolitique caractérisé par un rapport molaire de la silice à l'alumine d'au moins 12 et un indice de contrainte dans la gamme de 1 à 12 et la récupération d'un produit de condensation constitué dudit produit de type alcool.

2. Le procédé de la revendication 1, dans lequel ledit produit de type alcool contient 4 à 9 atomes de carbone et ledit composé insaturé contient 3 à 8 atomes de carbone.

3. Le procédé de la revendication 1 ou 2, dans lequel ledit produit de type alcool contient 5 atomes de carbone et ledit composé insaturé contient 4 atomes de carbone.

4. Le procédé de l'une quelconque des revendications précédentes, dans lequel ledit produit de type alcool est constitué de 3-méthylbuténols et de 3-méthylbutanediols et ledit composé insaturé est l'isobutène.

5. Le procédé de l'une quelconque des revendications précédentes, dans lequel ledit composé insaturé a un atome d'hydrogène qui est fixé en alpha de la liaison éthylénique carbone-carbone.

6. Le procédé de l'une quelconque des revendications précédentes, dans lequel ladite température est entre 20°C et 200°C, de préférence entre 50°C et 150°C.

7. Le procédé de l'une quelconque des revendications précédentes, dans lequel le formaldéhyde est sous forme d'une solution aqueuse.

8. Le procédé de l'une quelconque des revendications précédentes, dans lequel ledit aluminosilicate cristallin zéolitique est la ZSM—5, la ZSM—11 ou la ZSM—23.

9. Le procédé de l'une quelconque des revendications précédentes, dans lequel ledit aluminosilicate cristallin zéolitique est mélangé à un de ses liants.

10. Le procédé de l'une quelconque des revendications précédentes, dans lequel ladite réaction s'effectue dans un milieu solvant inerte.

11. Le procédé de l'une quelconque des revendications précédentes, dans lequel ladite réaction est effectuée à des pressions comprises entre l'atmosphérique et une supra-atmosphérique.

## Patentansprüche

1. Verfahren zur Herstellung eines Alkohol-Produkts, das 3 bis 25 Kohlenstoffatome enthält und ein Alkenol und ein Alkandiol umfaßt, bei dem eine Reaktion zwischen Formaldehyd und einer aliphatischen monoolefinisch ungesättigten Verbindung, die von 2 bis 24 Kohlenstoffatome enthält, bei einer Temperatur, die 250°C nicht überschreitet, in Gegenwart von Wasser und einem Katalysator mit einem kristallinen Alumosilicat-Zeolith bewirkt wird, der durch ein molares Verhältnis von Siliciumdioxid zu Aluminiumoxid von wenigstens 12 und einen Grenzwertindex (constraint index) im Bereich von 1 bis 12 gekennzeichnet ist, und bei dem ein Kondensationsprodukt gewonnen wird, das das genannte Alkohol-Produkt bildet.

2. Verfahren nach Anspruch 1, bei dem das genannte Alkohol-Produkt von 4 bis 9 Kohlenstoffatome enthält, und die genannte ungesättigte Verbindung von 3 bis 8 Kohlenstoffatome enthält.

3. Verfahren nach Anspruch 1 oder 2, bei dem das genannte Alkohol-Produkt 5 Kohlenstoffatome enthält, und die genannte ungesättigte Verbindung 4 Kohlenstoffatome enthält.

4. Verfahren nach einem beliebigen der vorangehenden Ansprüche, bei dem das genannte Alkohol-Produkt aus 3-Methylbutenolen und 3-Methylbutandiolen besteht und die genannte ungesättigte Verbindung Isobuten ist.

5. Verfahren nach einem beliebigen der vorangehenden Ansprüche, bei dem die genannte ungesättigte Verbindung ein Wasserstoffatom aufweist, das sich in Alpha-Stellung zu der ethylenischen Bindung zwischen 2 Kohlenstoffatomen befindet.

6. Verfahren nach einem beliebigen der vorangehenden Ansprüche, bei dem die genannte Temperatur zwischen 20°C und 200°C liegt, und vorzugsweise zwischen 50°C und 150°C.

7. Verfahren nach einem beliebigen der vorangehenden Ansprüche, bei dem der Formaldehyd in Form einer wäßrigen Lösung vorliegt.

8. Verfahren nach einem beliebigen der vorangehenden Ansprüche, bei dem der genannte kristalline Alumosilicat-Zeolith ZSM—5, ZSM—11 oder ZSM—23 ist.

9. Verfahren nach einem beliebigen der vorangehenden Ansprüche, bei dem der genannte kristalline Alumosilicat-Zeolith mit einem Binder für diesen Zeolith vermischt ist.

10. Verfahren nach einem beliebigen der vorangehenden Ansprüche, bei dem die genannte Reaktion im Medium eines inerten Lösungsmittels abläuft.

11. Verfahren nach einem beliebigen der vorangehenden Ansprüche, bei dem die genannte Reaktion bei Drucken von Atmosphärendruck bis überatmosphärischem Druck durchgeführt wird.